Europäisches Patentamt

European Patent Office

Office européen des brevets

(11) Publication number: **0 248 583 B1**

# EUROPEAN PATENT SPECIFICATION

(45) Date of publication of patent specification: **26.01.94** (51) Int. Cl.5: **A61K 37/66**

(21) Application number: **87304642.9**

(22) Date of filing: **26.05.87**

The file contains technical information submitted after the application was filed and not included in this specification

(54) **Treatment of basal cell carcinoma intralesionally with recombinant human alpha interferon.**

(30) Priority: **27.05.86 US 866644**

(43) Date of publication of application:
**09.12.87 Bulletin 87/50**

(45) Publication of the grant of the patent:
**26.01.94 Bulletin 94/04**

(84) Designated Contracting States:
**AT BE CH DE ES FR GB GR IT LI LU NL SE**

(56) References cited:
**EP-A- 0 077 063**
**EP-A- 0 233 629**
**EP-A- 0 281 299**

**Genetic Technology News,vol. 6, no. 10, 1986, page 5, H. Greenway: "But alpha interferon alanone destroys one type of tumor"**

**Journal of the American Academy of Dermatology, vol. 15 no. 3, September 1986, pages 437-443, H.T. Greenway et al.:"Treatment of basal cell carcinoma with intralesional interferon"**

**Biological Abstracts, vol. 82, no.9, 1986, page AB-643, ref. no. 84508, Philadelphia, US; H.**

**Morita et al.: "OKT 6-positive cells and lymphocyte subsets within skin tumors before and after therapy with local injection of interferon-a"**

**The Lancet, May 1981, pp. 1025-1027**

(73) Proprietor: **SCHERING CORPORATION**
**2000 Galloping Hill Road**
**Kenilworth New Jersey 07033(US)**

(72) Inventor: **Tanner, Daniel Jay**
**1401 55th Street**
**Brooklyn New York 11219(US)**
Inventor: **Peets, Edwin Arnold**
**160 W. 96th Street**
**New York New York 10025(US)**
Inventor: **Smiles, Kenneth Albert**
**44 Hawthorne Lane**
**East Windsor New Jersey 08520(US)**

(74) Representative: **Ritter, Stephen David et al**
**Mathys & Squire**
**10 Fleet Street**
**London EC4Y 1AY (GB)**

## Description

This invention relates to the preparation of pharmaceutical compositions for treating basal cell carcinoma. The compositions comprise recombinant human alpha interferon and are used by administering the interferon directly into the carcinoma lesion, i.e. intralesionally.

Basal cell carcinomas are the most common cutaneous neoplasms found in humans. The majority of the 500,000 new cases of nonmelanoma skin cancers each year are basal cell carcinomas.

Basal cell carcinomas exist in a variety of clinical and histological forms such as nodular-ulcerative, superficial, pigmented, morphea-like, fibroepithelioma and nevoid syndrome. Present treatment methods include various surgical techniques such as electrodesiccation and curettage, excision, cryosurgery and irradiation. Cure rates for the surgical techniques are generally greater than 95%. Despite the high cure rates effected by surgical techniques, non-surgical methods of therapy are generally thought to be more desirable.

Various efforts have been made to treat cancers by injecting interferon directly into the lesion. For example, Ikeda, Gan to Kagaku Ryoho, 12(4), 936-942 (1985) used recombinant interferon A to treat various malignant skin tumors and achieved low cure rates. None of the tumors treated were stated to be basal cell carcinomas. Sikora, K., Editor, Interferon and Cancer, Ikic, D., Intralesional Therapy, 169-181, Plenum, N.Y. (1983) used unpurified human leukocyte interferon to treat basal cell carcinoma patients intralesionally. Ikic did not use a purified interferon material, but used a material containing a mixture of leukocyte interferon components and non-interferon impurities.

Interferons are a family of proteins which exhibit antiviral activity agains certain viruses and anticancer activity agains certain cancers. There are three types of interferons; alpha or leukocyte interferon, beta or fibroblast interferon and gamma or immune interferon. Human alpha interferon is a naturally occuring mixture of at least eleven components including those designated alpha-1 interferon and alpha-2 interferon. Human alpha interferon exhibiting biological properties similar to those of naturally occurring human leukocyte interferon can be made by recombinant methods.

A number of alpha interferon species or components are known and are usually designated by a numeral after the Greek letter alpha, and all are contemplated for use in this invention. Thus, the species designated human alpha-1 interferon and human alpha-2 interferon (sometimes called human alpha-2 interferon which includes human alpha-2a and human alpha-2b interferon; USAN: Interferon Alfa-2 including Interferon alfa-2a and Interferon Alfa-2b) are contemplated, with human alpha-2 interferon preferred. Interferon alfa-2 can be produced in bacteria using recombinant techniques as disclosed in Rubenstein, Biochem. Biophys. Acta, 695, 5-16 (1982). In addition, interferon alfa-2 may be prepared by recombinant-DNA methods disclosed by Nagata et al., Nature, 284, 316-320 (1980), European Patent 32,134 and U.S. Patent 4,289,690. Various alpha-2-interferon species are disclosed in U.S. Patent 4,503,035.

According to the present invention there is provided the use of recombinant human interferon alpha-2b in the preparation of a pharmaceutical composition for treating human basal cell carcinoma comprising a sufficient amount of recombinant human interferon alpha-2b to be effective against basal cell carcinoma and a pharmceutically acceptable carrier therefor.

In use the compositions are administered intralesionally (by injection) to a patient in need of such treatment in an amount containing sufficient of said recombinant human interferon alpha-2b to be effective as an anti tumour agent.

For intralesional administration, liquid injectable pharmaceutically acceptable compositions are used. Such compositions can, for example, be prepared by diluting freeze dried hIFN-α2b with sterile preservative free water to produce an isotonic solution containing the appropriate concentration of interferon. Other injectable compositions using saline, aqueous dextrose, glycerol, ethanol and the like, to thereby form a solution or suspension for injection can also be used. If desired, minor amounts of nontoxic auxiliary substances such as wetting or emulsifying agents, preservatives, pH buffering agents and the like, for example, sodium acetate or sorbitan monolaurate, can be incorporated into the compositions. Actual methods of preparing such dosage forms are known, or will be apparent, to those skilled in this art; see for example, Remington's Pharmaceutical Sciences, Mack Publishing Company, Easton, P.A., 15th Edition, 1975. The amount of hIFN-α2b administered is critical only to the extent that it is effective for the therapeutic purpose. The quantity in the composition or formulation administered will, in any event, be an amount effective to achieve an effect against basal cell carcinoma in the subject being treated.

The amount of hIFN-α2b in a 0.15 ml. injectable dosage is about $1.5 \times 10^6$ I.U. (International Units). One "International Unit" is 0.5 nanogram.

In clinical tests to determine the effect of hIFN-α2b on basal cell carcinoma, the hIFN-α2b was administered in doses of $1.5 \times 10^6$ I.U. three days a week for three weeks, i.e. $13.5 \times 10^6$ I.U. total.

Although these doses and the regimen described were beneficial, they should be considered only guidelines and the attending clinician will determine, in his or her judgement, an appropriate dosage and regimen, using the patient's age and condition as well as the severity of the basal cell carcinoma.

The following illustrates the effects of treating patients having basal cell carcinoma with intralesionally administered interferon alfa-2b.

## PATIENTS AND METHODS

### Patients

Eight patients, six males and two females, each with one primary basal cell carcinoma of the nodular (3 patients) or superficial (5 patients) type were included in the study. The lesions, which varied in size from 7 x 6 mm. to 14 x 12 mm., were located on the back (5), shoulder (1), arm (1) and neck (1). The diagnosis of nodular or superficial basal cell carcinoma was confirmed by incisional biopsy at least one week prior to the initiation of treatment.

Each patient was in good health and elected to be treated with interferon alfa-2b rather than undergo other ablative or surgical procedures.

### Laboratory Test

Pretreatment laboratory tests included a complete blood count, a chemistry profile including renal and liver function tests, and a urinalysis. White blood cell counts also were taken 24 hours after the first treatment and the complete chemistry and hematology tests were repeated weekly during the treatment and one week after the end of treatment.

### Treatment

Treatments were conducted with freeze-dried human recombinant alpha-2b interferon which was in vials. The interferon in the vials was diluted with sterile, preservative-free water to produce an isotonic solution containing sufficient interferon concentration so that 0.15 ml of solution contained $1.5 \times 10^6$ International Units (IU). Each lesion was injected with 0.15 ml of alpha-2b interferon with a 30-gauge needle on a tuberculin syringe. The needle was inserted tangentially into the lesion with care being taken to inject the entire dose intralesionally. The procedure was repeated for a total of three injections per week for three weeks. Thus, each lesion was injected with a total of $13.5 \times 10^6$ I.U.

Patients were evaluated during the treatment for clinical response and side effects. Evaluations were continued at one, four and eight weeks after completion of treatment. Systemic side effects (i.e. flu-like symptoms) were treated and controlled with oral acetaminophen.

### Response Criteria

Excisional bioposy was performed on lesions of each of the eight patients eight weeks following completion of the treatment with alpha-2b interferon. The excisional biopsies were grossly serially sectioned at 2 mm. intervals, embedded in paraffin and then serially sectioned at 4 microns. All histopathologic slides were stained with hematoxylin and eosin. Clinical responses were measured during treatment and follow-up visits through evaluation of changes in lesion size, erythema and, in the case of nodular lesions, percentage of flattening.

The following Table I shows the results of treatment of basal cell carcinoma with recombinant human alpha-2 interferon.

TABLE I

| Results of Treating Basal Cell Carcinoma with Interferon Alfa-2b. | | | | | | | |
|------|-----|-----|---------------------------------|------------------------------------|-------------------------------------|----------------------------------------------|----------------------------------|
| Case | Age | Sex | Type of Basal Cell Carcinoma | Pre Treatment Lesion Size (mm) | Post Treatment Lesion Size (mm) | %Lesion Flattening (nodular lesions) only | Follow-up Excisional Biopsy |
| 1 | 59 | M | s | 8x8 | 6x3 | ----- | Ntn |
| 3 | 63 | M | s | 8x5 | 0 | ----- | Ntn |
| 5 | 50 | F | s | 14x12 | 3x2 | ----- | Ntn |
| 7 | 51 | M | s | 11x8 | 6x4 | ----- | Ntn |
| 8 | 58 | M | s | 12x10 | 6x4 | ----- | Ntn |
| 2 | 52 | M | n | 7x6 | 5x4 | 100 | Ntn |
| 4 | 50 | F | n | 9x8 | 6x3 | 100 | Ntn |
| 6 | 48 | M | n | 11x6 | 6x4 | 100 | Ntn |
| Abbreviations: s = superficial; n = nodular; Ntn = no tumor noted. | | | | | | | |

As is apparent from the data in Table I, all the basal cell carcinomas were eliminated. This was confirmed in the following histopathologic study.

Pretreatment incisional biopsies had confirmed the diagnosis of basal cell carcinoma in each patient. Histopathologic findings in the excisional biopsies were performed after completion of the injections and were reviewed; in these post-treatment biopsies two initial slides with multiple serial sections were evaluated. To ensure that the biopsy specimen was free of even microscopic foci of basal cell carcinoma, 10 slides with additional deep serial sections were examined. No basal cell carcinoma was found in any of the sections of the excisional biopsies.

The sites where the basal cell carcinomas had been treated demonstrated ectasia of vessels in the papillary dermis, perivascular lymphocytic and histiocytic accumulations, and sometimes lymphocyte exocytosis, civatte body formation and incontinence of pigment. No "tumor" stroma, necrotic basal cell carcinoma, polymorphonuclear neutrophil leukocytes, transformed lymphocytes or eosinophils were found.

SIDE EFFECTS

No life-threatening or serious side effects were found as shown in the following Table II.

## TABLE II

### Side Effects when Treating Basal Cell Carcinoma with Interferon alfa-2b.

| Patient No. | 1 | 2 | 3 | 4 | 5 | 6 | 7 | 8 | Total |
|---|---|---|---|---|---|---|---|---|---|
| Fever (after first dose) |  | + | + | + | + |  | + |  | 6 |
| Fever (after other doses) |  |  | + | + |  | + |  |  | 3 |
| Malaise | + |  |  | + | + | + | + |  | 5 |
| Itching (at site) |  | + |  | + |  | + |  |  | 3 |
| Lightheadedness |  |  | + |  |  |  |  |  | 1 |
| Pain (at site) |  |  |  | + |  |  |  |  | 1 |
| Muscle aches |  |  |  | + |  |  | + |  | 2 |
| Joint aches |  |  |  | + |  | + |  |  | 2 |
| Depressive mood |  |  |  | + |  |  |  |  | 1 |
| Headache |  |  |  |  | + | + |  |  | 2 |
| Abdominal discomfort |  |  |  |  |  | + |  |  | 1 |
| Chills |  |  |  |  |  | + |  |  | 1 |

All of the above side-effects were mild or moderate, except that some of the malaise symptoms were severe.

As the data in Table II indicate fever was the most common side effect followed by malaise. All side effects were transient and reversible, and all patients were able to complete the treatment regimen.

The only laboratory results which were abnormal were diminished white blood cell counts in three patients. In each case the white blood cell count returned to normal before the program was completed.

The side effects observed were those to be expected with the use of intralesional administration of interferon. It was noted that the side effects were more frequent at the first injection and became fewer as the program progressed.

The results of the above-described program show that intralesional injection of interferon alfa-2b is a safe, effective treatment for basal cell carcinoma, yielding excellent cosmetic results.

**Claims**

1. Use of recombinant human interferon alpha-2b in the preparation of a pharmaceutical composition for treating human basal cell carcinoma comprising a sufficient amount of recombinant human interferon alpha-2b to be effective against basal cell carcinoma and a pharmaceutically acceptable carrier therefor.

**Patentansprüche**

1. Verwendung von rekombinantem Human-Interferon-alpha-2b zur Herstellung einer pharmazeutischen Zusammensetzung zum Behandeln von menschlichem Basalzellenkarzinom, welche eine Menge an rekombinantem Human-Interferon-alpha-2b, die ausreichend ist, um gegen Basalzellenkarzinom wirksam zu sein, und einen pharmazeutisch annehmbaren Träger dafür enthält.

**Revendications**

1. Utilisation de l'interféron alpha-2b humain de recombinaison dans la préparation d'une composition pharmaceutique pour le traitement de carcinomes de cellules basales humaines, composition comprenant une quantité suffisante d'interféron alpha-2b humain de recombinaison, pour être efficace contre les carcinomes de cellules basales, et un porteur de cet interféron acceptable d'un point de vue pharmaceutique.